# EUROPEAN PATENT APPLICATION

(11) **EP 2 508 223 A1**
(43) Date of publication of application: **10.10.2012**
(21) Application number: 12305412.4
(22) Date of filing: 06.04.2012
(51) Int. Cl.: A61M 25/10, A61B 1/00, A61B 17/3207

(54) **Balloon catheter with drug delivery probe**

(30) Priority: 08.04.2011 US 201161473519 P; 30.03.2012 US 201213436389
(71) Applicant: Sanovas, Inc., Sausalito, CA 94965 (US)
(72) Inventor: Gerrans, Lawrence J., San Anselmo, CA 94960 (US); Gunday, Erhan H., Great Neck, NY 11021 (US)
(74) Representative: Denjean, Eric

(57) **Abstract**

A balloon catheter (20) with delivery probe is provided. The catheter (22) has a balloon (24), a first lumen (26) and a probe (28) movably disposed in the first lumen of said catheter. The probe has a lumen (34) for delivering a diagnostic and/or therapeutic agent to tissue. The catheter has a third lumen through which fluid is supplied to the balloon. The catheter with delivery probe may be inserted into a bodily cavity such that the distal end of the probe extends out of an opening (30) in the catheter. When the balloon is inflated the probe is captured between an outer wall of the balloon and the tissue so that therapeutic and/or diagnostic agent may be delivered to the tissue through the probe lumen via the distal end of the probe. Furthermore, two additional balloons (224, 202) disposed distally and proximally of the balloon to create a chamber.

## Description

### FIELD OF THE INVENTION

The present invention relates to methods and systems for delivering therapeutic and/or diagnostic agents to specific cellular locations within and adjacent to bodily tissues and cavities. More specifically, the invention relates to a method and system of delivering diagnostic and/or therapeutic agents to bodily tissues and cavities via a balloon catheter with a drug delivery probe.

### BACKGROUND OF THE INVENTION

In diagnosing and treating diseases of various body cavities and organs, it is necessary to deliver diagnostic and/or therapeutic agents to the organs at specified locations. The most common routes of drug delivery include non-invasive peroral (through the mouth), topical (skin), transmucosal (nasal, buccal/sublingual, vaginal, ocular and rectal) and inhalation. However, many therapeutic and diagnostic agents in general may not be delivered using these routes because the agents may be susceptible to enzymatic degradation or cannot be absorbed into the systemic circulation efficiently due to molecular size and charge issues, and thus, will not be therapeutically effective. For this reason, many such drugs have to be delivered by injection.

There are several known problems associated with the injection process. One of such problems is undesirable extravasation of the diagnostic or therapeutic agents into tissue, which is particularly prevalent with intravenously injected agents. Extravasation generally refers to leakage of fluids out of a container, and more specifically refers to leakage of intravenous drugs from a vein into surrounding tissues, resulting in injury to the tissues. Once the intravenous extravasation has occurred, damage can continue for months and involve nerves, tendons and joints. If treatment is delayed, surgical debridement, skin grafting, and even amputation have been known to be the unfortunate consequences.

Occurrence of extravasation is possible with all intravenuous drugs, but it is a particularly significant problem with cytoxic drugs used for treatment of cancer (i.e. during chemotherapy).

Chemotherapy is the general term for any treatment involving the use of chemical agents to stop cancer cells from growing. Chemotherapy can eliminate cancer cells at sites great distances from the original cancer. As a result, chemotherapy is considered a systemic treatment. More than half of all people diagnosed with cancer receive chemotherapy. A chemotherapy regimen (a treatment plan and schedule) usually includes drugs to fight cancer plus drugs to help support completion of the cancer treatment.

Chemotherapy can be administered through a vein, injected into a body cavity, or delivered orally in the form of a pill, depending on which drug is used. Chemotherapy works by destroying cancer cells. Unfortunately, it cannot tell the difference between a cancer cell and some healthy cells. Thus, chemotherapy often eliminates not only the fast-growing cancer cells, but also other fast-growing cells in the body, including hair and blood cells. Some cancer cells grow slowly while others grow rapidly. As a result, different types of chemotherapy drugs target the growth patterns of specific types of cancer cells.

Each chemotherapy drug works differently and is effective at a specific time in a life cycle of the cell it targets. Brachytherapy, sometimes called seed implantation, is an outpatient procedure used in the treatment of different kinds of cancer. The radioactive "seeds" are carefully placed inside of the cancerous tissue and positioned in a manner that will attack the cancer most efficiently. The radioactive seeds are about the size of a grain of rice, and give off radiation that travels only a few millimeters to kill nearby cancer cells. There are two different kinds of brachytherapy: permanent, when the seeds remain inside the body, and temporary, when the seeds are inside of the body and are then removed. With permanent implants (e.g. prostate), the radioactivity of the seeds typically decays with time.

The other type of chemotherapy is when cytotoxic agents are delivered intravenously. Veins of people receiving chemotherapy are often fragile, mobile, and difficult to cannulate. Patients who receive chemotherapy at the same site as radiotherapy may experience a reactivation of skin toxicity known as a "recall" phenomenon. Patients who have had previous radiation therapy at the site of injection may develop severe local reactions from extravasated cytotoxic drugs. Cytotoxic drugs also have the potential to cause cutaneous abnormalities in areas that have been damaged previously by radiation, even in areas that are distant from the injection site. Patients who have had an extravasation and receive further chemotherapy in a different site may experience an exacerbation of tissue damage in the original site.

Furthermore, areas of previous surgery where the underlying tissue is likely to be fibrosed and toughened dramatically present an increased risk of extravasation. Radical mastectomy, axillary surgery or lymph node dissection may impair circulation in a particular limb. This reduces venous flow and may allow intravenous solutions to pool and leak around the site of cannulation.

Some chemotherapy drugs often never reach the tumors they are intended to treat because the blood vessels feeding the tumors are abnormal. A tumor's capillaries (small blood vessels that directly deliver oxygen and nutrients to cancer cells) can be irregularly shaped, being excessively thin in some areas and forming thick, snarly clumps in others. These malformations create a turbulent, uneven blood flow, so that too much blood goes to one region of the tumor, and too little to another. In addition, the capillary endothelial cells lining the inner surface of tumor capillaries, normally a smooth, tightly-packed sheet, have gaps between them, causing vessel leakiness.

The systemic and intravenous side effects of chemotherapy coupled with the limited effect of systemic administration due to abnormal characteristics of tumor blood vessels have given the scientific community pause, in searching for more direct, localized and biologic solutions. Accordingly, the oncology literature has become increasingly populated with articles espousing prospective benefits and positive outcomes of intra-tumoral chemotherapy. A direct administration of cytotoxic drugs such as Mytomycin, Mytomycin-C, Bleomycin, Fluorouracil, Mitoxantrone, Cisplatin, and Avastin in endobronchial intra-tumoral chemotherapy has been done experimentally via direct injection of the agent into the endobronchial tumor. In these cases, the tumor was reported to have died and been subsequently removed.

However, while some experimental uses of the localized delivery of cytotoxic drugs have been attempted, there has been little implementation of such drug delivery in practice, possibly due to numerous problems associated with such delivery. First, it is often necessary to deliver cytotoxic drugs to remote and not easily accessible blood vessels and other lumens within body organs, such as lungs. It is also important to be able to deliver defined doses of the cytotoxic substances because such substances are often very expensive or are capable of causing serious harm if delivered in excess. Moreover, the existing methods lack the ability to contain the cytotoxic agent and/or radiation therapy and mitigate collateral damage to non-affected anatomy and structures.

Several devices have been proposed for a targeted delivery of drugs to internal bodily cavities. For example, U.S. Pat. No. 5,397,307 to Goodin discloses a catheter having a pair of longitudinally spaced balloons with a drug delivery region between the balloons. The proximal balloon seals a vessel while the distal balloon is contoured such that when inflated, drug injected into the drug delivery region flows slowly past adjacent tissue and bathes the treated site with medicament. U.S. Pat. No. 7,611,484 to Wellman et al. discloses a multi-balloon catheter designed for treatment of deceased blood vessels, and specifically lesions in the blood vessels. The catheter includes a pair of end balloons that, when inflated, isolate the deceased region of the blood vessel. The catheter further includes a middle balloon having an outer wall with a plurality of micro-needles that enable the therapeutic agents to be injected into the blood vessel wall.

While the above described catheter devices are useful for delivering the drugs to a specific target site, these systems are not particularly efficient at infusing the relevant biological material with the drug. Instead, the catheter may need to remain in place for an unnecessarily long period of time while the infusion of the drug into the biological material is allowed to take place. This is undesirable, especially in applications such as pulmonology, where the patient's respiratory passage has been somewhat restricted by the device. Further, this can result in some of the agent never being infused into the targeted material and instead remaining in the cavity and, after the balloon catheter is removed, subsequently migrating to other undesired portions of the body.

What is desired, therefore, is a balloon catheter system for delivering therapeutic and/or diagnostic agents to bodily tissues, tumors, and other biological materials that can locally deliver the agent to a specific target site. What is further desired is a balloon catheter system for delivering therapeutic and/or diagnostic agents that facilitates the infusion of the drug into surrounding bodily tissues, tumors, and other biological materials.

### SUMMARY OF THE INVENTION

Accordingly, it is an object of the present invention to provide a balloon catheter system that can deliver therapeutic and/or diagnostic agents to bodily tissues, tumors, and other biological materials from within bodily cavities.

It is a further object of the present invention to provide a balloon catheter system that can target specific areas for the delivery of therapeutic and/or diagnostic agents to bodily tissues, tumors, and other biological materials.

It is yet another object of the present invention to provide a balloon catheter system that stimulates flow of blood cells and thereby facilitates infusion of therapeutic and/or diagnostic agents into surrounding bodily tissues, tumors, and other biological materials.

It is another object of the present invention to provide a balloon catheter system for delivering therapeutic and/or diagnostic agents to bodily tissues, tumors, and other biological materials that permits the passage of bodily fluids through the system.

It is yet another object of the present invention to provide a balloon catheter system for delivering therapeutic and/or diagnostic agents to bodily tissues, tumors, and other biological material that provides visualization within the bodily cavity.

In order to overcome the deficiencies of the prior art and to achieve at least some of the objects and advantages listed, the invention comprises a balloon catheter for delivering a therapeutic and/or diagnostic agent to tissue, comprising a catheter having a balloon and a first lumen, and a probe movably disposed in the first lumen of the catheter. The probe has a second lumen for delivering a diagnostic and/or therapeutic agent to tissue. The catheter has an outer wall with an opening therethrough, through which the probe passes through the outer wall. The catheter also has a third lumen through which fluid is supplied to the balloon to retain the probe between the balloon and the tissue.

In some embodiments, the balloon catheter further comprises a fluid source that supplies fluid to the third lumen. In some of those embodiments, the fluid source comprises an electro-pneumatic pump. In certain of those embodiments, the pump supplies fluid to the balloon in pulsed fashion to repeatedly deflate and inflate said balloon. In certain embodiments, the fluid source further comprises a vacuum source that evacuates fluid from said balloon. In certain advantageous embodiments, the fluid is a gas.

In certain embodiments, the balloon has an outer wall comprising an abrasive surface for abrading tissue.

In some embodiments, the balloon catheter further comprises an imaging system movably disposed in said catheter for viewing the tissue.

In certain embodiments, the balloon comprises at least one imaging marker.

In some embodiments, the catheter further comprises a fourth lumen through which bodily fluids pass.

In certain advantageous embodiments, the delivery probe has a distal tip having an arcuate shape.

In some embodiments, the agent is doxorubicin. In other embodiments, the agent is cisplatin, and the method further includes the step of supplying a second agent, the second agent being epinephrine. In further embodiments, the agent is 5-4 fluorouracil. In yet further embodiments, the agent is a combination of at least one therapeutic agent and at least one biomarker, and the method further includes the step of monitoring extravasation of the at least one therapeutic agent into tissue via the at least one biomarker. In some of these embodiments, the biomarker is a radio-opaque marker.

The invention also comprises a catheter with delivery probe having three balloons. The catheter has a first balloon, a second balloon disposed proximal to the first balloon, a third balloon disposed distal to the first balloon and a first lumen. There is a probe movably disposed in the first lumen of the catheter, the probe having a second lumen for delivering a diagnostic and/or therapeutic agent to tissue. The catheter has an outer wall with an opening therethrough between the first balloon and the third balloon, through which the probe passes from the first lumen through the outer wall. The catheter has a third lumen through which fluid is supplied to at least the first balloon to retain the probe between the first balloon and the tissue.

In some embodiments, a fluid source supplies fluid to the third lumen. In some of those embodiments, the fluid source comprises an electro-pneumatic pump. In certain of those embodiments, the pump supplies fluid to the first balloon in pulsed fashion to repeatedly deflate and inflate the first balloon. In certain embodiments, the fluid source further comprises a vacuum source that evacuates fluid from the first balloon. In certain advantageous embodiments, the fluid is a gas.

In certain embodiments, the catheter has a fourth lumen through which fluid is supplied to the second balloon and the third balloon to inflate the second balloon and the third balloon such that a chamber is created between the second balloon and the third balloon. In some of those embodiments, a fluid source supplies fluid to the third and fourth lumen.

The invention also comprises a method of delivering a therapeutic agent and/or diagnostic agent to tissue. The method comprises the steps of inserting a catheter into a bodily cavity, the catheter having an opening, and a balloon, moving a delivery probe including at least one delivery lumen through the catheter such that a distal end of the delivery probe extends out of the opening in the catheter, inflating the at least one balloon by supplying fluid thereto to capture the distal end of the delivery probe between an outer wall of the balloon and the tissue, and delivering a therapeutic and/or diagnostic agent through the at least one delivery lumen to the tissue via the distal end of the delivery probe.

In some embodiments, the method further comprises the step of repeatedly deflating and inflating the at least one balloon by supplying fluid to the balloon in a pulsed fashion such that the repeated deflation and inflation causes said therapeutic and/or diagnostic agent to spread on the tissue. In some of those embodiments, the step of inflating the balloon comprises supplying fluid to the balloon with an electro-pneumatic pump, and the step of repeatedly deflating and inflating the balloon is controlled by the electro-pneumatic pump based at least partially on an established volume change or frequency.

In certain embodiments, the balloon has an outer wall with an abrasive surface; and the step of inflating said balloon further comprises abrading tissue in the bodily cavity with the abrasive surface.

In some embodiments, the method further comprises the step of using an imaging device disposed in said catheter to visualize tissue in the bodily cavity. In certain of these embodiments, the imaging device is used to position the drug delivery probe adjacent the tissue.

In some embodiments, the delivery probe comprises a first delivery lumen and a second delivery lumen. In some of those embodiments, a first therapeutic and/or diagnostic agent is delivered through said first delivery lumen to the tissue via the distal end of the delivery probe and a second therapeutic and/or diagnostic agent is delivered through said second delivery lumen to the tissue via the distal end of the delivery probe.

In certain advantageous embodiments of the method, the balloon is a first balloon and the catheter further comprises a second balloon and a third balloon. The second balloon is proximal to the first balloon, the third balloon is distal to the first balloon, and the opening is between the second and third balloons.

Other objects of the invention and its particular features and advantages will become more apparent from consideration of the following drawings and accompanying detailed description.

### BRIEF DESCRIPTION OF THE DRAWINGS

FIG. 1A is perspective view a balloon catheter with delivery probe of the present invention.

FIG. 1B is a cross-sectional view along line 1-1 of the probe of FIG. 1 A.

FIG. 2 is side, partially schematic view of the balloon catheter with delivery probe of FIG. 1A including a fluid source.

FIG. 3 is a cross-sectional view along line 3-3 of the balloon catheter of FIG. 1A.

FIG. 4 is a partially exposed, side view of a balloon catheter of the present invention within a bodily cavity.

FIGS. 5A-5B are cross-sectional views of the balloon catheter of FlG 4.

FIGS. 6A-6C are partially exposed, side views of a balloon catheter with delivery probe of the present invention being operated within a bodily cavity.

FIGS. 7A-7E are side views of a balloon catheter with delivery probe of the present invention being operated within a bodily cavity.

FIG. 8 is a perspective view of a delivery probe used in the present invention.

FIG. 9 is a side view of a catheter with delivery probe of the present invention including an imaging device therein.

FIG. 10 is a partially exposed, isometric view of a balloon catheter with delivery probe including an imaging device therein within a bodily cavity.

FIG. 11 is a schematic view of a delivery mechanism of the probe of FIG. 1.

### DETAILED DESCRIPTION OF THE INVENTION

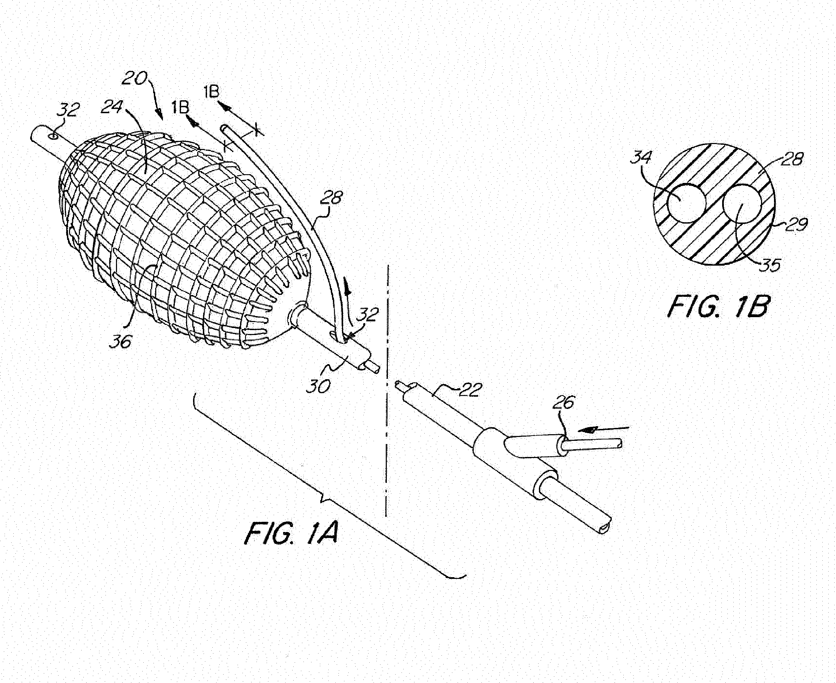

The basic components of one embodiment of a balloon catheter with drug delivery probe in accordance with the invention are illustrated in FIG. 1. As used in the description, the terms "top," "bottom," "above," "below," "over," "under," "above," "beneath," "on top," "underneath," "up," "down," "upper," "lower," "front," "rear," "back," "forward" and "backward" refer to the objects referenced when in the orientation illustrated in the drawings, which orientation is not necessary for achieving the objects of the invention.

As shown in FIGS. 1A and 1B, the balloon catheter with drug delivery probe (20) includes a catheter (22) having a balloon (24) and a first lumen (26). The catheter (22) has an outer wall (30) with an opening (32) therethrough that connects to the first lumen (26). A probe (28) is movably disposed in the first lumen (26) of said catheter (22) and able to pass from the first lumen (26) through said outer wall (30) via opening (32). The probe (28) comprises at least one delivery lumen (34) for accommodating therapeutic and/or diagnostic agents. The catheter (22) also includes a third lumen (not shown) through which fluid is supplied to the balloon (24).

The catheter (22) may have any suitable diameter and length depending on a particular application, and may be flexible, rigid or semi rigid. The catheter (22) may be made with any commercially available material that is flexible enough to allow the catheter to be safely inserted through the available opening of a bodily cavity such that it will bend instead of puncturing the walls of the cavity, and at the same time is rigid enough such as it will maintain its shape as it is passed alongside and/or through the available opening of the bodily cavity. In an advantageous embodiment, the catheter (22) consists of a coil wire made of any suitable material, such as stainless steel, and a coating made of polyethylene.

The balloon (24) may be made of latex, Yulex, polyethylene, nylon or other suitable material, and may come in a variety of sizes and diameters, which allow the balloon catheter to be used in bodily cavities of various diameters and dimensions, such as large and small bronchial branches, sinuses, and blood vessels, having different types of tumors and tissues to be treated.

In other advantageous embodiments, the balloon (24) includes radiopaque markings and calibrations that aid direct visualization via endoscopic imaging and in-direct visualization via Radiography, MRI, CT, Ultrasound and/or other modalities of imaging known in the art.

The balloon (24) has a wall with outer surface that acts an abrasion surface. In some embodiments, the outer surface (36) may comprise a fiber mesh affixed to the surface of the balloon (24) during the molding process, which produces outwardly-facing protrusions on the surface of the balloon (24) that assist in gripping and or optimize the abrasion capability of the balloon to the surrounding tissue. The fiber mesh may be made of lycra, polyurethane, composite springs, or other appropriate material. In other embodiments, dimensional surface structures or inflatable sinuses that are encapsulated in the surface substrate of the balloon (24) may be used to produce the surface protrusions.

The abrading surface can be used to abrade bodily tissues, such as airway or vessel walls. The abrasion of the bodily tissues stimulates bleeding and instigates flow of white blood cells, i.e. leukocytes, out of the circulatory system towards the site of tissue damage. This process, together with the application of volumetric pressure or force to the abraded surface of the airway or the vessel wall to neutralize hemodynamic shear forces, perpetuates fluid extravasation processes and stimulates associated cellular absorption of the diagnostic and/or therapeutic agents into the adjacent tissues. The textured outer surface (36) of the balloon (24) can also act as a gripping surface for attachment to bodily tissues, such as blood vessel walls, to anchor the balloon (24) at a target tissue site.

The balloon catheter (20) may include one or more openings (32) positioned on either side of balloon (24). In addition to facilitating delivery of the diagnostic and/or therapeutic agents via the drug delivery probe (28), the openings (32) may be used to evacuate the agents and/or other fluids that may be present at the delivery site using negative pressure via suction/vacuum. In various other embodiments, the openings (32) are intended to enable cyclical delivery and evacuation of the diagnostic and/or therapeutic agents and various other fluids instantly, sequentially, intermittently and/or constantly over designated time intervals.

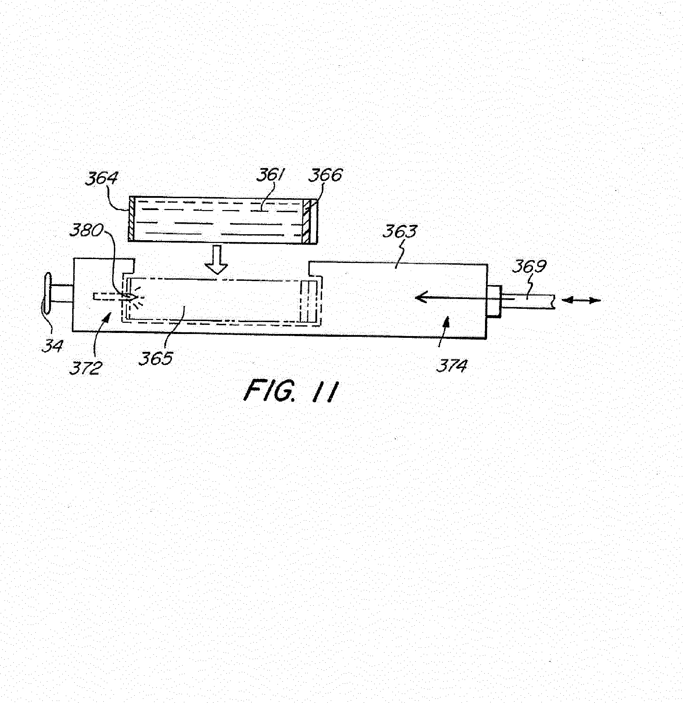

The therapeutic and/or diagnostic agent can be delivered to the delivery lumen (34) of probe (28) via any suitable mechanism. In one advantageous embodiment (shown in FIG. 11), the therapeutic and/or diagnostic agent is contained in a drug capsule (361) adapted to be positioned into a delivery apparatus (363). The drug capsule (361) is prefilled with the agent and is sealed at a distal end by a piercable membrane (364) and at a proximal end by a slidable piston (366). The capsule (361) can be made out of any suitable material, and preferably is transparent such that the amount of the agent delivered can be monitored. The size of the drug capsule (361) and the amount of the agent it can hold can be variable depending on a particular application. For example, the capsule (361) can be filled with the amount of the agent to be delivered plus the amount needed to prime the drug delivery lumen (34) of the probe (28).

The drug capsule (361) fits into a capsule compartment (365) of the delivery apparatus (363), which is connected to the delivery lumen (34) at a distal end (372) and is connected to a fluid source at a proximal end (374) via a lumen (369). The fluid source (368) can be the same pump that is used to inflate the inner balloon or can be a separate pump. The distal end (372) of the capsule compartment has a needle (380) or any other suitable piercing device that functions to pierce the membrane (364) of the capsule (361). The proximal end (374) of the capsule compartment has an actuation mechanism adapted to actuate the piston (366) of the capsule (361). The capsule compartment (365) is preferably made out of transparent material such that the location of the piston can be determined and therefore the amount of the agent delivered can be observed and monitored.

The capsule (361) filled with the therapeutic and/or diagnostic agent is first positioned into the capsule compartment (365) of the delivery apparatus (363), such that the membrane (364) is pierced by the needle (380) located at the distal end (372) of the capsule compartment (365) to allow the agent to exit out towards the delivery lumen (34). Once the drug capsule (361) is securely positioned inside the capsule compartment (365), the actuation mechanism actuates the piston (366) such that it moves towards the distal end of the capsule (361), ejecting the therapeutic and/or diagnostic agent out of the capsule into the delivery lumen (34) of the probe (28). If the agent to be delivered to tissue is in gaseous form, the delivery apparatus (363) can further include a valve (not shown) positioned at the distal end of the apparatus before the connection to the drug delivery lumen (34). The valve controls how much gaseous agent is delivered to the lumen (34), as well as the delivery time.

The actuator mechanism of the delivery apparatus (363) can be a pneumatic cylinder, into which fluid is supplied by the fluid source, wherein fluid pressure pushes the piston (366) forward, ejecting the agent out of the capsule (361). In other embodiments, the actuator mechanism can be an electrical motor, e.g. a stepper motor or a servo motor. The actuator mechanism (363) is connected to a controller that controls the quantity of the agent to be delivered and the delivery time period. The controller can be pre-programmed to deliver the exact quantity of drug over the exact amount of time, or it can be operated manually by the user during the procedure.

The piston (366) is preferably provided with a sensor, e.g. a magnetic sensor, optical or mechanical encoder, or any other suitable type of sensor, so that the position of the piston can be detected and communicated to the controller, which then determines how much drug has been delivered. A pressure transducer can also be provided at the distal end of the delivery apparatus (363) for measuring pressure in the drug delivery lumen (34) and reporting it to the controller that regulates the drug delivery rate and detects any problems that may arise.

It should be noted that other embodiments of the drug delivery mechanism can be used without departing from the spirit of the present invention. The drug capsule prefilled with the agent to be delivered can be primed at any location along the probe (28).

In certain embodiments, the probe (28) has multiple lumens to supply therapeutic agents to the target tissue, which allows for delivery of multiple agents separately, as may be desired when using two different pharmaceuticals that should not be mixed until just before being extravasated into bodily tissue. For example, as shown in FIG. 1B, the probe (28) can include two delivery lumens (34) and (35), each supplying a different agent via a separate opening in the probe. Likewise, one may need to deliver one medicinal agent at the beginning of the procedure, and another medicinal agent at a later time during the procedure. Furthermore, one may wish to deliver a second agent at a slightly different location than the first agent, which can be accomplished by providing two separate exit holes in the probe (28), such as, for example, at the distal end of the probe (28) or an outer wall (29), and delivering each agent to tissue adjacent to each of those exit holes. Alternatively, one could deliver a first agent via lumen (34) when the probe (28) is protruding through a first opening in the catheter, such as opening (32) located proximally of the balloon (24), then move the probe (28) to and through a second opening in the catheter, such as opening (32) located distally of the balloon (24), and then deliver a second agent via lumen (35).

The probe (28) may deliver an appropriate inert dye or contrast media (radioactive, polarized, florescent, temperature sensitive, etc.) in addition to the drug allowing the drug infusion rate and the amount of drug infused into the tissue to be monitored, quantified, displayed and reported by a controller by capturing sequential video frames under different illumination conditions (UV, IR, polarized, color filters, etc.).

In a preferred embodiment, the probe (28) deploys a contrast media such as a radiopaque dye, or imaging contrast solution, along with the diagnostic and/or therapeutic agent so as to visually identify the target tissue and/or discern the requisite volumetric pressure, force, temperature, frequency and/or time to achieve efficacious delivery of the agent to desired depths of penetration at the intended treatment site when the intended area is visualized via direct and/or indirect visualization apparatus such as Endoscopic, Radiographic, Ultrasonic, MRI, CT or other imaging modalities known in the art.

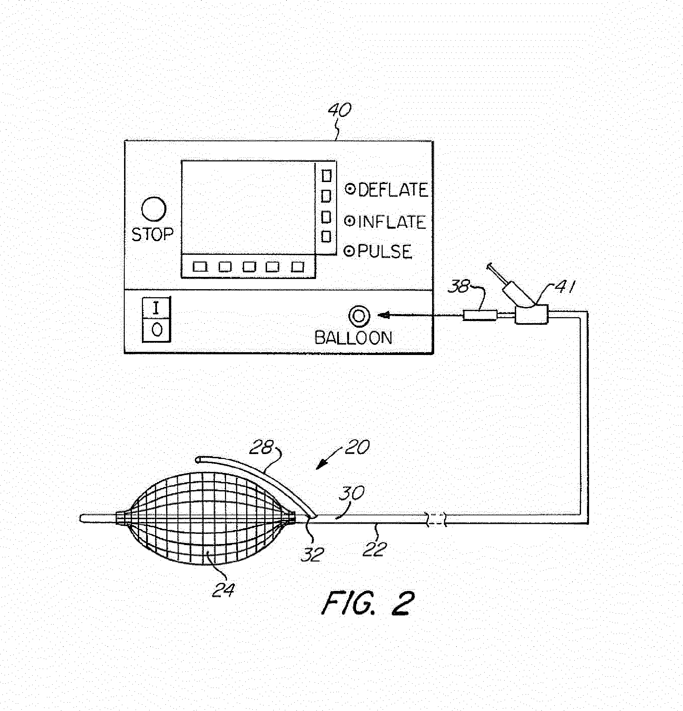

As shown in FIG. 2, in certain embodiments, the proximal portions of the device (20) include a port (38) for connection of the balloon (24) to a fluid source (40), such as a manually actuated inflation apparatus or an electro-pneumatic pump, through which the balloon (24) can be expanded. Any suitable fluid source may be used in accordance with the present invention. In the preferred embodiment shown in FIG. 2, the fluid source (24) is an electro-pneumatic pump having controls on the front thereof, from which a physician or assistant can control the system (as well as a remote control unit), such as that disclosed in U.S. Patent Application No. 2010/0121270 by Gunday et al., the specification of which is hereby incorporated by reference herein in its entirety.

A connection mechanism (38) may be provided with any suitable connector or a plurality of connectors, such as a luer connector, for connection to the manually actuated expansion apparatus or inflation pump (40). The manually actuated inflation apparatus and/or pump (40) supplies a fluid, such as a gas, liquid, or mixture thereof, to the balloon (24) via a lumen, or a plurality of lumens provided in the elongated catheter shaft. Any suitable type of a manually actuated inflation apparatus or pump may be used in accordance with the present invention. In a preferred embodiment, a PhysioSense pump is used, which may control the amount of inflation, expansion and/or activation of the expansion apparatus based on actual bodily lumen measurements and confirmed by pressure reading as well to ensure appropriate expansion.

The port (38) may be connected to a lumen breakout V junction (41) to facilitate the introduction the delivery probe (28). The breakout junction may also be a Y junction to bring out a separate, inner or auxiliary lumen (not shown) of the catheter (22) to a suitable connector. Additionally, a V or Y junction (41) could also include a shut-off valve for stopping the balloon (24) from deflating.

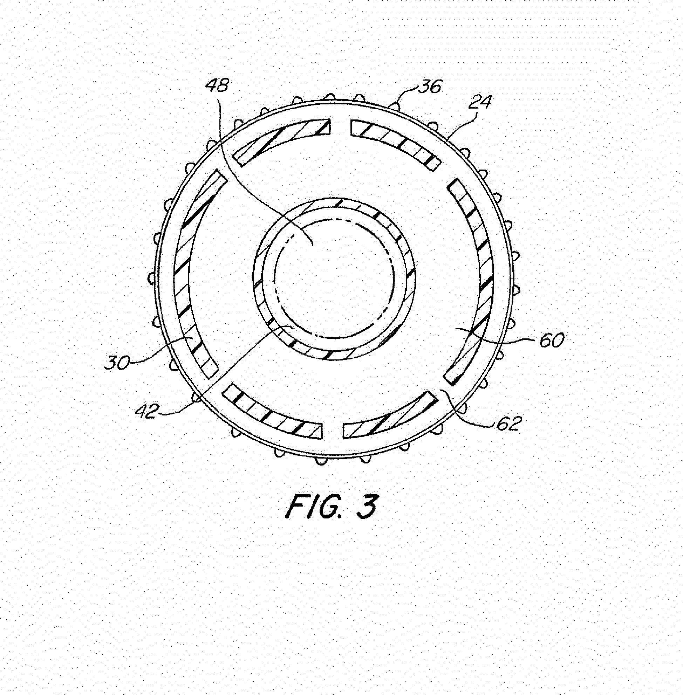

FIG. 3 illustrates a cross section of the balloon catheter apparatus (20) at the distal end corresponding to the location of the balloon (24). In certain embodiments, an outer lumen (60) extends though the catheter body for supplying fluid from fluid source (40) to the balloon (24) in order to inflate the balloon, which is shown in a deflated state. A distal end of the outer lumen terminates at the balloon (24) and a proximal end of the outer lumen is connected to the fluid source (40). The balloon (24) may be inflated and deflated through holes (62) provided in the catheter's outer walls (30) that exit into the bladder of the balloon (24). These outer lumens (60) are blocked at the distal end of the balloon (24) so that air intended for inflation and deflation will not escape.

Additionally, the device includes an inner lumen (42) that can be used as a means for accurately positioning the balloon catheter (20), as it can be used as a conduit for a guide wire (48) when inserting the deflated balloon catheter (20) into the bodily cavity. The inner lumen (42) may extend the entire length of the catheter (22). In certain embodiments, the inner lumen (42) of the catheter (22) extends through the catheter tip and is open at the distal end. In this case, the inner lumen (42) serves as a passageway that allows the body fluids or air to move freely through the catheter construct while it remains in a cavity.

It should be noted that the catheter construct may include a plurality of inner lumens (42) that can be used to deliver any number of things to assist insertion and positioning of the construct within the bodily cavity. For example, additional lumens may be provided for introduction of an imaging and/or illumination system to facilitate the movement of the catheter construct through the cavities and to assure precise positioning of the guide wire (48) at the target site.

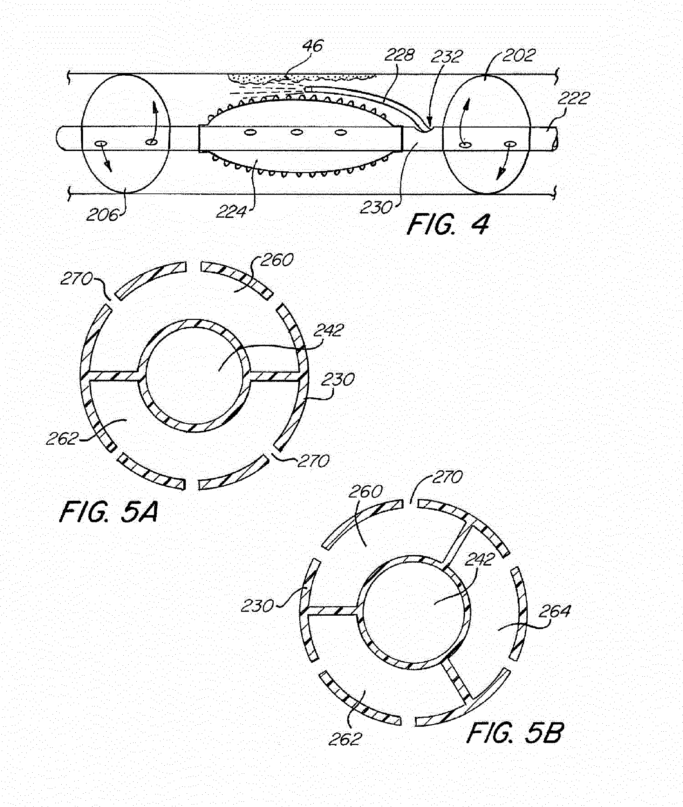

FIG 4 shows an advantageous embodiment of a balloon catheter with delivery probe within a bodily cavity. The catheter (222) includes a first balloon (224), a second balloon (202) proximal to the first balloon (224) and a third balloon (206) distal to the first balloon (224). The catheter (222) also includes a first lumen (not shown). The catheter (222) has an outer wall (230) with an opening (232) therethrough that connects to the first lumen. A probe (228) is movably disposed in the first lumen of said catheter (222) and able to pass from the first lumen through the outer wall (230) via opening (232). The probe (228) comprises at least one delivery lumen (not shown) for accommodating therapeutic and/or diagnostic agents.

Employing separate proximal and distal balloon segments in this way serves several purposes. First, one is able to inflate the proximal and distal balloon segments (202, 206) to an amount appropriate to hold the catheter (222) steady where target tissue is located while the center balloon (224) is cyclically inflated and deflated to facilitate the distribution and extravasation of diagnostic and/or therapeutic agents. By doing so, one can prevent the agent from escaping into the bodily cavity, and instead, can capture the excess agent for easy removal.

FIGS. 5A and 5B are cross-sectional views of the catheter (222) showing how multiple lumens may be used to inflate and deflate the three balloons (224, 202, 206). As noted above, the inner lumen (242) is used for air bypass and/or a guide wire conduit. The lower lumen (262) has inflation/deflation hole (270) in the catheter walls only at the position along the length of the catheter (222) where the center balloon (224) is located, while the upper lumen (260) contains inflation/deflation holes (270) only at the position along the length of the catheter where the proximal and distal balloons (202, 206) are located. It should be noted that the proximal and distal balloon segments (202, 206) can also be inflated/deflated independently from each other by further separating the outer lumens (260, 262) to include an additional lumen (264), and positioning the inflation/deflation holes (270) at the appropriate locations along the length of catheter (222). Likewise, additional balloon segments could be added, which could each similarly be inflated independently from the others by increasing the number of lumens and adding a separate termination at the proximal end of the catheter.

Additionally, in some of these multiple-balloon embodiments, imaging markers (e.g., radio opaque rings), can be located at or near the ends of each balloon segment in order to facilitate the use of certain imaging modalities to assist with the precise positioning of the balloons.

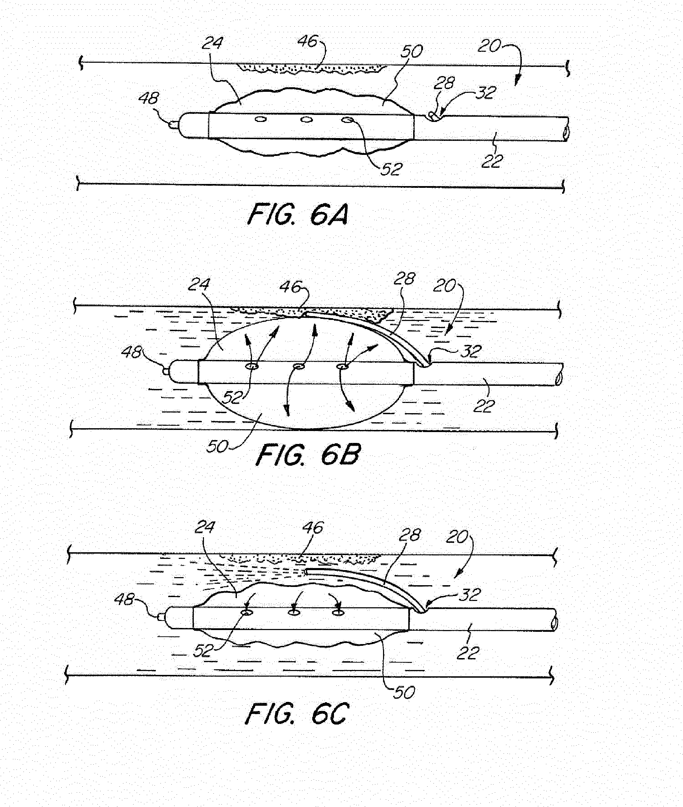

The operation of a balloon catheter with drug delivery probe (20) can generally be described with reference to FIGS. 6A-6C. Referring first to FIG. 6A, after a visual inspection via an endoscope, x-ray, and/or ultrasound, a balloon catheter (20) is selected, and the deflated device is inserted into position in a bodily cavity. This may be accomplished by using the working channel of an endoscope or, as previously noted, along a guide wire (48) that is previously inserted into the body and inserting the proximal end of the guide wire (48) which is outside the body into the inner lumen of the catheter. The catheter (20) is advanced over the guide wire until the distal end of the guide wire (48) extends out of a lumen of the catheter (22). The appropriate positioning of the device (20) may be determined by aligning markings on the distal and/or proximal end of the guide wire with the distal and/or proximal end of the catheter's inner lumen (42). The step may be monitored under fluoroscopy to ensure that the balloon or expansion apparatus has not moved out of position. An imaging system may also be employed during this procedure through inner channels provided in the catheter.

A delivery probe (28) having at least one lumen for delivering a diagnostic and/or therapeutic agent is inserted into a lumen in the catheter body (22) and passed through an opening (32) in the outer wall of the catheter (22). The device (20) is connected to a pump, at which time the pump may determine the type of balloon catheter (20) that has been inserted.

Referring next to FIG. 6B, the probe (28) is positioned near target tissue (46) within the bodily cavity and substantially above or below a surface of the balloon (24). In some cases, it is advantageous to use imaging means to facilitate this step, as described in further detail below. Simultaneously, or after positioning the probe, fluid is supplied to the inflation chamber (50) via the inflation lumen (60) in the catheter (22) through the plurality of openings (52) to inflate the balloon (24). It should be noted that, although a plurality of openings (52) in the catheter (22) is illustrated in FIGS. 6A-6C, one opening is sufficient to supply fluid to inflate the balloon (24).

As the balloon (24) inflates, probe (28) is substantially retained against the target tissue (46). Once, the probe (28) is appropriately positioned, diagnostic and/or therapeutic agent(s) are delivered and released from delivery lumens therein.

As shown in FIG. 6C, after diagnostic or therapeutic agent is released from probe (28) and onto surfaces of the surrounding bodily cavity and target tissue (46), the balloon (24) is deflated and the catheter device (20) may be removed from the bodily cavity.

Optionally, when a pulse button on the pump is pressed, balloon (24) may be deflated and inflated in a cyclical fashion, based either on parameters that were entered by the user, or on default parameters selected by the pump, which are based on the characteristics of the particular balloon and diameter and/or density measurements made by the system. In this way, the pulse mode of the pump causes the balloon (24) to pulsate according to a desired frequency or change in volume within the balloon, producing a periodically recurring increase and decrease in balloon size. By pulsating the balloon (24) in this way, one can facilitate extravasation of the agent(s) into the surrounding tissue. Additionally, in embodiments in which the outer surface of the balloon includes an abrasive surface, using this pulsation either before or during the delivery of a therapeutic agent can stimulate absorption of the agent by the surrounding tissue (46).

Any of various agents useful in therapeutic application can be delivered in the above described manner. For example, the agent may comprise one or more chemical or biological drugs with useful pharmacological properties, as well as any other medicaments or other substances with medicinal or other therapeutic uses. Such agents may be synthetic or natural, so long as they have an advantageous therapeutic effect that can obtained by delivering the agent to a target site. In certain embodiments, agents particularly useful for chemotherapies, radiation therapies, or immunotherapies are delivered as described above.

In some advantageous embodiments, a cytotoxic substance or other agent useful for chemotherapy is delivered to a target site via the balloon catheter system with delivery probe of the present invention. For example, in some cases, the catheter system is used to deliver a chemical agent that affects cell division or DNA synthesis. Such agents include, for example, alkylating antineoplastic agents, such as cisplatin, carboplatin, oxaliplatin, mechlorethamine, carmustine, cyclophosphamide, chlorambucil, ifosfamide, busulfan, treosulfan, melphalan hydrochloride, thiotepa, and dacarbazine; antimetabolites, such as azathioprine, mercaptopurine, thioguanine, fludarabine, pentostatin, cladribine, fluorouracil, floxuridine, cytosine arabinoside, gemcitabine, methotrexate, pemetrexed, and raltitrexed; anthracenedione antineoplastic agents, such as mitoxantrone; anthracyclines, such dactinomycin, daunorubicin, doxorubicin, epirubicin, idarubicin, valrubicin, aclarubicin, and bleomycin; plant alkaloids and terpenoids, such as noscapine, vincristine, vinblastine, vinorelbine, vindesine, podophyllotoxin, paclitaxel, and docetaxel; topoisomerase inhibitors, such as irinotecan, topotecan, amsacrine, etoposide, etoposide phosphate, and teniposide; and other agents with similar mechanisms of action, such as mitomycin C.

Other such agents include those that target molecular abnormalities, including tyrosine kinase inhibitors, such as crizotinib, gefitinib, erlotinib hydrochloride, imatinib, and imatinib mesilate. Still other such agents include those that modulate tumor cell behavior without actually attacking the cells, such as may be employed for hormone treatments. Indeed, any drug known to be efficacious in treating cancerous cells, such as streptozotocin or diltiazem augment taxol, may be employed.

In certain advantageous embodiments, a biological response modifier or other biological agent useful for immunotherapy is delivered to a target site via the balloon catheter with delivery probe. Such agents, which are often cytokines, may be a recombinant, synthetic, or natural preparation. These biological agents may include, for example, interferons, such as alpha-interferons and beta-interferons; interleukins, such as aldesleukin; colony-stimulating factors, such as filgrastim, sargramostim, epoetin, and oprelvekin; monoclonal antibodies, such as edrecolomab, rituximab, trastuzemab, gemtuzumab, alemtuzumab, nimotuzumab, cetuximab, bevacizumab, ibritumomab, panitumumab, and tositumomab; cancer vaccines; gene therapies; and non-specific immunomodulating agents. Any biologic known to useful for immunotherapies, such as asparaginase, may be employed.

In some advantageous embodiments, the therapeutic agent is delivered in drug eluting microspheres, which can be used both to cause the embolization of blood vessels that supply undesirable tissues and to retain the drug in a localized area for a sustained period of time. For example, drug-eluting microspheres can be used to deliver a chemotherapeutic drug, such as doxorubicin, to a tumor. When the microspheres reach the target site, they will block vessels supplying the tumor, and this suppression of blood flow will lead to ischemia. Over time, the microspheres break down, and the drug will be absorbed by the tissue. As a result, not only is a localized sustained realease of the drug achieved, but the ischemia will also increase the effect of the drug on the tumor.

The above described delivery of therapeutic agents is also useful for radiation therapies, in which high-energy radiation is used to kill cancer cells and shrink tumors. One method of such therapy places radioactive material in the body near the cancer cells. Thus, in certain advantageous embodiments, a radioactive substance, such as a radiolabeled monoclonal antibody, is supplied via the balloon catheter with delivery probe and extravasated into nearby tissue as described below.

Various agents may also be employed to assist in making diagnostic observations or monitoring procedures. For example, in some advantageous embodiments, the above described system may be used to deliver a contrast agent that allows or improves visualization via one or imaging modalities, which can be used to image the extravasation of the agent into the surrounding tissues throughout the course of a procedure. Such agents may include, for example, radiocontrast agents, such as iodine or barium, to improve X-ray based imaging techniques; MRI contrast agents, such as gadolinium, to improve magnetic resonance imaging; and microbubble contrast agents, to improve ultrasound imaging.

In some advantageous embodiments, biomarkers are used together with a therapeutic agent to observe and monitor the extravasation of the agent into the surrounding tissues. In some of these advantageous embodiments, CF3PM & MTFN-1 fluorinated radio-opaque biomarkers are used. The biomarkers may be detected by various non-invasive imaging modalities, such as X-Ray, MRI, CT, ultrasound, spectroscopy, etc.

With the addition of an appropriate inert dye or contrast media (e.g., radioactive, polarized, florescent, temperature sensitive) to a drug to be extravasated, the drug infusion rate and the amount of drug infused into the tissue can be monitored, quantified, and recorded/displayed, such as, for example, by capturing and storing sequential video frames under different illumination conditions (UV, lR, polarized, color filters, etc.). Further, by deploying a contrast agent along with a therapeutic agent, one can visually identify the extravasation depths and/or discern the requisite volumetric pressure, force, temperature, frequency and/or time to achieve efficacious delivery of the therapeutic agent to the desired depth of penetration at the intended treatment site.

The balloon catheter with drug delivery probe of the present invention can also be used to supply various media, e.g. light based therapies, radiofrequency wave forms, thermal energies and temperatures, and pressured air, to modulate cellular response sufficient to achieve tumoral destruction and to alter cellular membrane integrity to facilitate extravasation of medicinal and/or diagnostic agents into bodily tissues.

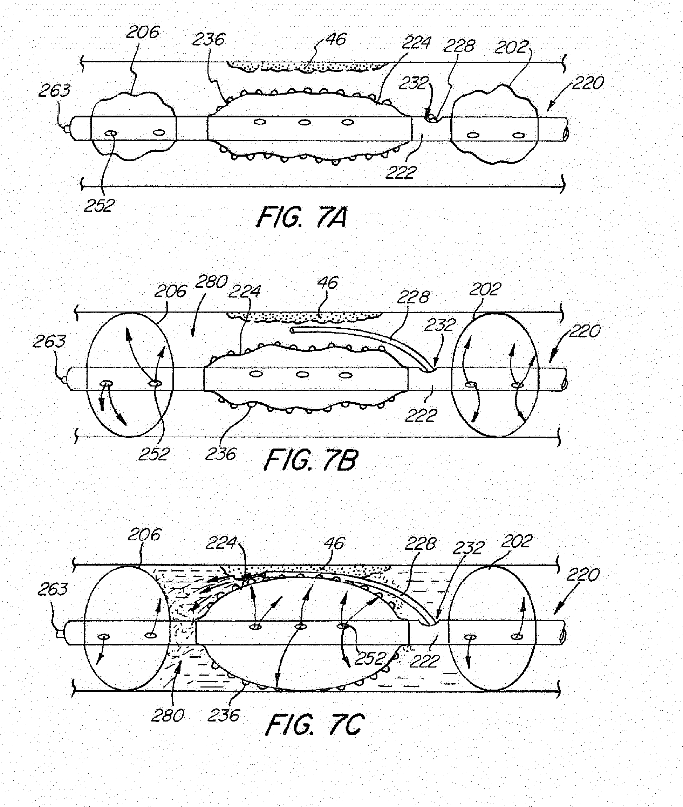

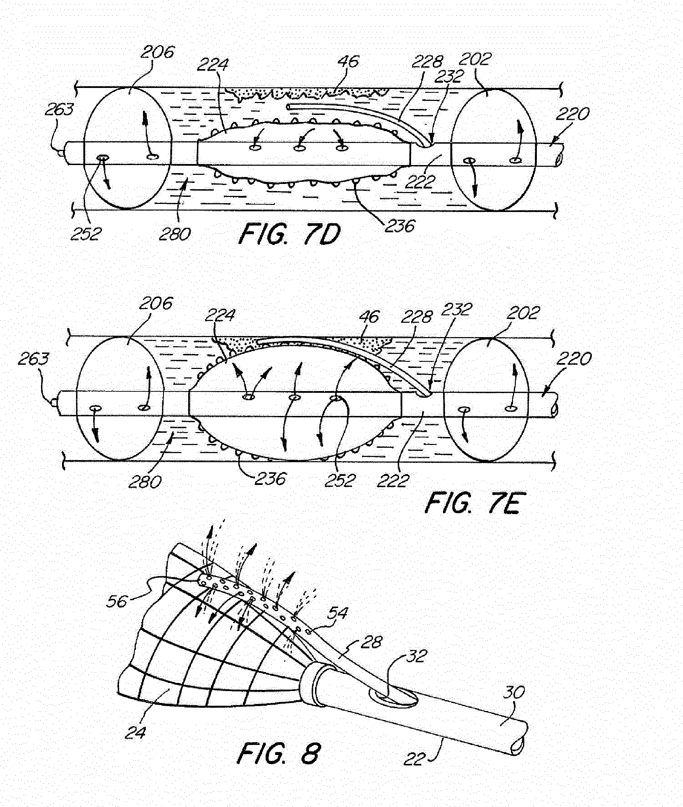

An advantageous method of operation, employing any of the above described agents with a three balloon catheter with drug delivery probe, can generally be described with reference to FIGS. 7A-7E. Referring first to FIG. 7A, after a visual inspection via an endoscope, x-ray, and/or ultrasound, a balloon catheter (220) is selected, and the deflated device is inserted into position in a bodily cavity. This may be accomplished by using the working channel of an endoscope or, as previously noted, along a guide wire (263). A delivery probe (228) having at least one lumen for delivering a diagnostic and/or therapeutic agent is inserted into a lumen in the catheter body (222) and passed through an opening (232) in the outer wall of the catheter (222). The device (220) is connected to a pump, at which time the pump may determine the type of balloon catheter (220) that has been inserted.

Referring next to FIG. 7B, fluid is supplied to the inflation chamber of the outer balloons (202, 206) via the inflation lumens (262, 264) and through the plurality of openings (252) to inflate the balloons (202, 206), until the balloons are in sufficient contact with the bodily cavity to seal the target tissue (46) within a delivery chamber (280). After the outer balloons (202, 206) are inflated, the probe (228) is positioned near target tissue (46) within the bodily cavity and substantially above or below a surface of the balloon (224) containing abrading members (236). It is advantageous to use an imaging means to facilitate this step, as described in further detail below. Fluid is then supplied to abrading balloon (224) via inflation lumen (260) and through openings (252) to inflate the balloon (224) until probe (228) is substantially retained against the target tissue (46).

As shown in FIG. 7C, once, the probe (228) is appropriately positioned and retained, diagnostic and/or therapeutic agent(s) are delivered and released from delivery lumens therein and onto surfaces of the surrounding bodily cavity and target tissue (46). Referring to FIGS. 7D-7E, the abrading balloon (224) is then deflated while outer balloons (202, 206) remained inflated, preventing the diagnostic and/or therapeutic agent from spreading outside the delivery chamber (280). Optionally, as described above, when a pulse button on the pump is pressed, the balloon (224) is deflated and inflated in a cyclical fashion such that the diagnostic and/or therapeutic agent is extravasated into the tissue.

Accordingly, the abrading surface of the balloon (224) repeatedly comes into contact with the target tissue (246) to create micro-impacts thereon. As the balloon is deflated and inflated, the abrading members (236) may promote compressive force exhaustion and abrasion to elicit decomposition of tissue or to facilitate infusion or absorption into target tissues (46). Any loose tissue or excess agent may be removed through opening (232) or another available lumen.

Now also referring back to FIGS. 4-5B, the balloon catheter with drug delivery probe (220) may further include inline pressure valves that enable the lumens (260, 262, 264) to be pressurized, while preventing unintended depressurization. The delivery chamber (280) is pressurized by inflating the central balloon (224) and/or by pressurizing the chamber (280) with pneumatic pressured air or by other means. In an advantageous embodiment, the diagnostic and/or therapeutic agent contains a radiopaque contrast that permits visualization such as, Radiography, MRI, CT, Ultrasound, Endoscopic and/or other means of visualization known in the art to image the extravasation of the agent into the surrounding tissues throughout the course of treatment. Once the agent has reached, and sufficiently saturated, the intended treatment site with localized delivery of the agent, the remaining agent is removed. The chamber is then irrigated, lavaged and suctioned to remove all remnant agent. The proximal and distal expansion apparatus (202, 206) are deflated, contracted and/or deactivated and the construct is pulled out of the body.

Turning now to FIG. 8, an embodiment of the delivery probe (28, 228) is illustrated. Although only one exit hole (54) is necessary, probe (28) contains a plurality of exit holes (54) at the distal end of the construct extending from the outer wall of the probe (28) to one or more delivery lumens contained therein. The exit holes (54) allow therapeutic or diagnostic agent supplied through the lumen of the delivery probe (28) to be delivered from the probe. Distributing exit holes (54) around the outer surface of the distal end of the probe (28) allows the agent(s) to be delivered without having to rotate the probe (28). The exit hole(s) may also be positioned at the distal tip (56) of the probe (28).

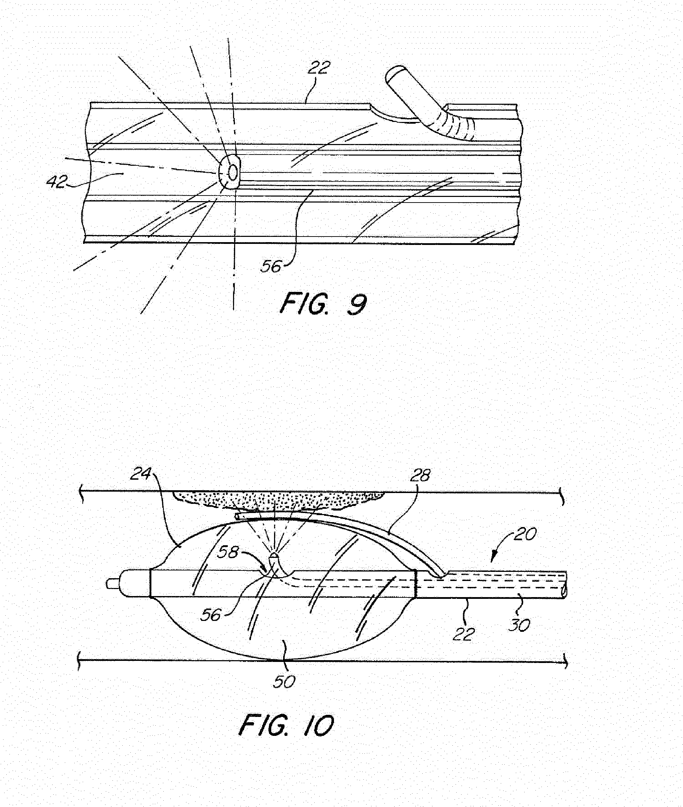

In some embodiments, as shown in FIGS. 9 and 10, a fiber optic image bundle (56) is introduced through the inner lumen (42) or any inflation or auxiliary lumen (not shown) to image the surrounding area. At the proximal end of the balloon catheter (20, 220) a junction (41) may provide access. The fiber optic image bundle can be made of an incoherent fiber bundle for illumination and a coherent imaging fiber bundle at the core, and a lens. Two separate bundles, one for illumination and the other for imaging, can also be used. At the distal end of the fiber optic bundle (56), the imaging coherent fibers are separated from illumination fibers (not shown) and interfaced to an image sensor, such as CMOS or CCD, through appropriate optics (not shown). Similarly, the illumination fibers are interfaced to a light source (not shown). It should be noted, however, that other sources of illumination, such as light emitting diodes, may also be employed. It should also be noted that the image sensor (CCD or CMOS available today in 2mm size) can be located at the tip of the imaging catheter assembly (not shown), eliminating the need for coherent imaging fiber bundle, thus increasing the image quality and reducing cost.

In this sort of way, the physician can be provided with illuminated light, non-thermal illuminated light, and direct visual feedback of the area ahead of the balloon (24), along the sides of the balloon, and/or behind the balloon. It is particularly advantageous for the catheter and/or the balloon to be made of a transparent or translucent (i.e., see-through) material so that the bundle (56) can provide images of the surrounding bodily cavity while it is maintained within the catheter (22) or within the inflation chamber (50) of a balloon (24). The transparent or translucent balloon allows the bundle (56) to provide imaging and/or illumination capabilities that facilitate positioning the delivery probe (28). As shown in FIG. 10, the catheter (22) may contain an opening (58) through the outer wall (30) of the catheter (22) within the balloon (24) such that the bundle (56) can pass through the opening (58) into the balloon chamber (50) for more direct imaging or lighting of the target tissue (46) and delivery probe (28). The imaging sensor and illumination optics possess the ability to be translated linearly or rotationally through and/or around the balloon (24), thereby allowing for 360° visualization of the treatment area.

In some advantageous embodiments, the distal end of the catheter (22, 222) includes a transparent membrane made out of any suitable material. The imaging device is extended through one of the lumens of the catheter to the membrane, which allows for visualization of the area ahead of the catheter (22, 222). In this way, the physician can be provided with illuminated light and direct visual feedback of the area ahead of the balloon catheter, along the sides of the balloons, and/or behind the balloons.

In other advantageous embodiments, the lumen of the catheter (22, 222), in which the imaging device is disposed, has an opening at a distal end, and the imaging device is extended out of the opening to visualize tissue in front of the balloon catheter device (20, 220). In this embodiment, the catheter (22, 222) can also be provided with a cleaning device at the distal tip for cleaning the imaging device. The cleaning device is made with any suitable type of material, such as textile bundle, and is affixed to an inner surface of the catheter (22, 222) adjacent to the opening at the distal end. The imaging device is cleaned by moving it back and forth through the textile bundle, thus wiping a lens of the imaging device.

In cases where holes (e.g. 58) are to be used or it is desirable to look back, the imaging devices come with a pre-shaped distal tip. This feature enables the distal tip to come out of the side holes (with the rotation of the image guide outside the body when the distal tip reaches the hole). If the three balloon catheter is deployed using a flexible or rigid endoscope then the imaging from the endoscope is also available at the proximal end.

It should be understood that the foregoing is illustrative and not limiting, and that obvious modifications may be made by those skilled in the art without departing from the spirit of the invention. Accordingly, reference should be made primarily to the accompanying claims, rather than the foregoing specification, to determine the scope of the invention.

## Claims

1. A balloon catheter for delivering a therapeutic and/or diagnostic agent to tissue, comprising:
a catheter having a balloon and a first lumen; and
a probe movably disposed in the first lumen of said catheter, said probe having a second lumen for delivering a diagnostic and/or therapeutic agent to tissue;
wherein said catheter has an outer wall with an opening therethrough, through which said probe passes through said outer wall; and
wherein said catheter has a third lumen through which fluid is supplied to said balloon to retain said probe between said balloon and said tissue.

2. The balloon catheter of claim 1, further comprising a fluid source that supplies fluid to the third lumen.

3. The balloon catheter of claim 1, wherein said fluid source comprises an electro-pneumatic pump.

4. The balloon catheter of claim 3, wherein said pump supplies fluid to the balloon in pulsed fashion to repeatedly deflate and inflate said balloon.

5. The balloon catheter of claim 4, wherein said fluid source further comprises a vacuum source that evacuates fluid from said balloon.

6. The balloon catheter of claim 1, wherein the balloon has an outer wall comprising an abrasive surface for abrading tissue.

7. The balloon catheter of claim 1, further comprising an imaging system movably disposed in said catheter for viewing the tissue.

8. The balloon catheter of claim 1, wherein said balloon comprises at least one imaging marker.

9. The balloon catheter of claim 1, wherein said catheter further comprises a fourth lumen through which bodily fluids pass.

10. The balloon catheter of clam 1, wherein the delivery probe has a distal tip having an arcuate shape.

11. A balloon catheter for delivering a therapeutic and/or diagnostic agent to tissue, comprising:
a catheter having a first balloon, a second balloon disposed proximal to said first balloon, a third balloon disposed distal to said first balloon, and a first lumen; and
a probe movably disposed in the first lumen of said catheter, said probe having a second lumen for delivering a diagnostic and/or therapeutic agent to tissue;
wherein said catheter has an outer wall with an opening therethrough between said first balloon and said third balloon, through which said probe passes from said first lumen through said outer wall; and
wherein said catheter has a third lumen through which fluid is supplied to at least said first balloon to retain said probe between said first balloon and said tissue.

12. The catheter of claim 11, further comprising a fluid source that supplies fluid to said third lumen.

13. The balloon catheter of claim 12, wherein said fluid source supplies fluid to said first balloon in pulsed fashion to repeatedly deflate and inflate said first balloon.

14. The catheter of claim 11, wherein said catheter has a fourth lumen through which fluid is supplied to said second balloon and said third balloon to inflate said second balloon and said third balloon such that a chamber is created between said second balloon and said third balloon.

15. The catheter of claim 14, further comprising a fluid source that supplies fluid to said third lumen and said fourth lumen.
